# EUROPEAN PATENT APPLICATION

(11) **EP 4 371 463 A1**
(43) Date of publication of application: **22.05.2024**
(21) Application number: 22842269.7
(22) Date of filing: 17.05.2022
(51) Int. Cl.: A61B 1/00, A61B 1/24, A61B 5/00, A61C 9/00, A61C 19/04

(54) **ORAL SCANNER SYSTEM**

(30) Priority: 15.07.2021 KR 20210092796
(71) Applicant: Osstemimplant Co., Ltd., Gangseo-gu Seoul 07789 (KR)
(72) Inventor: KIM, Eun Joong, Seoul 07064 (KR); CHOI, Hyun Ho, Seoul 07509 (KR); SHIN, Dong Wook, Seoul 07514 (KR); HONG, Ju Myung, Seoul 03687 (KR)
(74) Representative: Beck & Rössig European Patent Attorneys
(86) International application number: PCT/KR2022/007077
(87) International publication number: WO 2023/287006

(57) **Abstract**

An embodiment of the present disclosure provides an oral scanner system including: an oral scanner body which has an optical device and an imaging board disposed therein and is configured to receive power from an external power source or an internal battery; and an oral scanner calibration device configured to support the oral scanner body and calibrate the optical device using a pattern plate provided therein, wherein, upon insertion of the oral scanner calibration device into the oral scanner body, the oral scanner calibration device is electrically connected to the oral scanner body, and power is supplied to the oral scanner calibration device.

## Description

### [Technical Field]

The present disclosure relates to an oral scanner system, and more particularly, to an oral scanner system for calibrating an oral scanner by utilizing a pattern plate or the like to improve accuracy of the oral scanner.

### [Background Art]

In the process of producing a dental prosthesis, impression taking is an important process for producing an accurate prosthesis. Conventionally, an impression is taken using an impression material, and a prosthesis is produced based on a plaster model produced after the impression is taken, but there is a disadvantage that there is an error due to deformation of the impression material and the use of plaster.

Accordingly, intraoral structures such as teeth or gums are measured using an optical 3D imaging device using an optical 3D scanner, and a dental prosthesis is produced using a computer-aided design (CAD)/computer-aided manufacturing (CAM) system. In particular, in recent years, a stereo vision method using images obtained by two or more imaging devices has been applied to an oral scanner.

In the oral scanner using the stereo vision method, an image of one point of an oral cavity is captured by two or more imaging devices to obtain two or more pieces of image data, and 3D distance information is obtained based on the two or more pieces of image data, and thus calibration is frequently required for the oral scanner in order to obtain accurate 3D model data. For this reason, a calibration tool is typically provided in the form of a cradle as a separate accessory in the 3D oral scanner.

### [Disclosure]

### [Technical Problem]

The present disclosure is directed to providing an oral scanner system that can supply power to an oral scanner calibration device during calibration of an oral scanner even when a separate power source is not provided in the oral scanner calibration device.

### [Technical Solution]

One aspect of the present disclosure provides an oral scanner system including: an oral scanner body which has an optical device and an imaging board disposed therein and is configured to receive power from an external power source or an internal battery; and an oral scanner calibration device configured to support the oral scanner body and calibrate the optical device using a pattern plate provided therein, wherein, upon insertion of the oral scanner calibration device into the oral scanner body, the oral scanner calibration device is electrically connected to the oral scanner body, and power is supplied to the oral scanner calibration device.

In one embodiment, the oral scanner body may include a first substrate electrically connected to the external power source or the internal battery and a connector part mounted on the first substrate, and the oral scanner calibration device may include a second substrate and a joining part mounted on the second substrate.

In one embodiment, the oral scanner calibration device may include a casing in which an insertion part, into which the oral scanner body is inserted, is formed.

In one embodiment, upon insertion of the oral scanner body into the insertion part, the connector part and the joining part may be electrically connected.

In one embodiment, the connector part may be formed of a groove including a conductive line, the joining part may be formed of a pin including a conductive line, and upon the insertion of the oral scanner body into the insertion part, the pin may be inserted into the groove.

In one embodiment, the connector part may be positioned at an end of the oral scanner body, and the joining part may be positioned at the insertion part.

In one embodiment, the insertion part may be formed to correspond to a shape of the end of the oral scanner body.

In one embodiment, the oral scanner calibration device may include a driving part configured to be driven to change one or more of an angle and a distance of the pattern plate relative to the optical device.

In one embodiment, the oral scanner calibration device may include a controller configured to control operation performance of the driving part.

In one embodiment, the oral scanner calibration device may include a communication part configured to communicate with an image processing device or the oral scanner body and transmit a control signal to the controller.

### [Advantageous Effects]

According to one aspect of the present disclosure, since it is possible to apply power of an oral scanner body to an oral scanner calibration device by inserting the oral scanner body into the oral scanner calibration device, it is efficient in terms of energy, and size reduction of the oral scanner calibration device can be achieved.

The advantageous effects of the present disclosure are not limited to the above-mentioned advantageous effects and should be understood as including all effects inferable from configurations of the present disclosure described in the detailed description or claims of the present disclosure.

### [Description of Drawings]

FIG. 1 is a perspective view of an oral scanner system according to one embodiment of the present disclosure.
FIGS. 2 and 3 are exploded perspective views of an oral scanner calibration device according to one embodiment of the present disclosure.
FIG. 4 is a lateral cross-sectional view of the oral scanner calibration device according to one embodiment of the present disclosure.
FIG. 5 shows enlarged views of the oral scanner system according to one embodiment of the present disclosure.
FIG. 6 is a schematic diagram of the oral scanner system according to one embodiment of the present disclosure.
FIG. 7 is a schematic diagram of the oral scanner system according to another embodiment of the present disclosure.

### [Modes of the Invention]

Hereinafter, the present disclosure will be described with reference to the accompanying drawings. However, the present disclosure may be implemented in various different forms and thus is not limited to the embodiments described herein. Also, in the drawings, parts irrelevant to the description have been omitted to clearly describe the present disclosure, and like parts are denoted by like reference numerals throughout the specification.

Throughout the specification, when a certain part is described as being "connected" to another part, this includes not only the case in which the certain part is "directly connected" to the other part, but also the case in which the certain part is "indirectly connected" to the other part with another member disposed therebetween. Also, when a certain part is described as "including" a certain element, the certain part may further include another element instead of excluding other elements unless particularly described otherwise.

Terms including ordinals such as "first" and "second" used herein may be used to describe various elements or steps, but the corresponding elements or steps should not be limited by ordinals. The terms including ordinals should be construed as only being used for the purpose of distinguishing one element or step from another element or step.

Hereinafter, embodiments of the present disclosure will be described in detail with reference to the accompanying drawings.

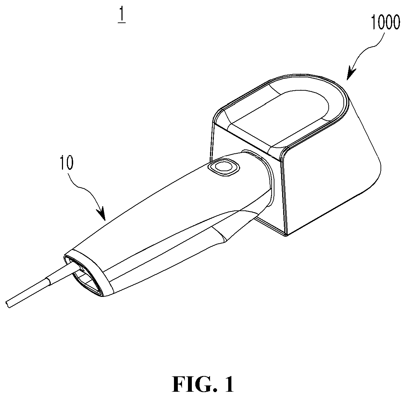

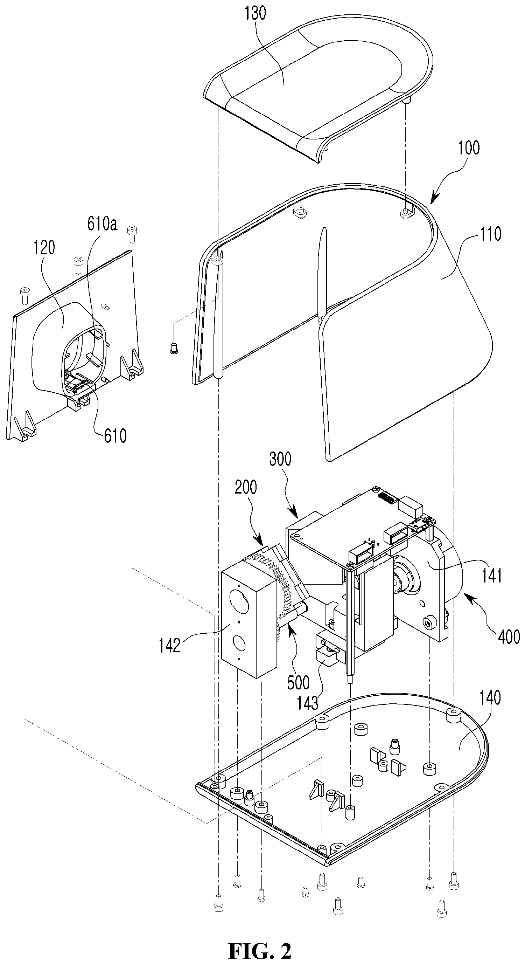

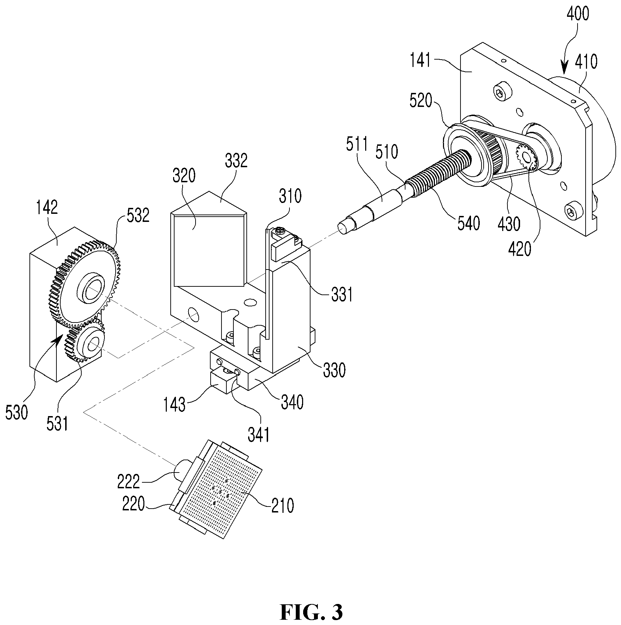

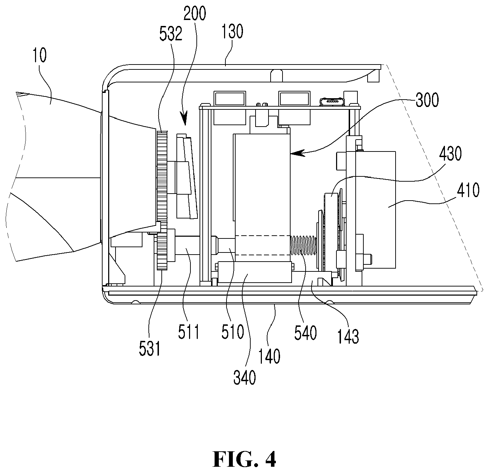

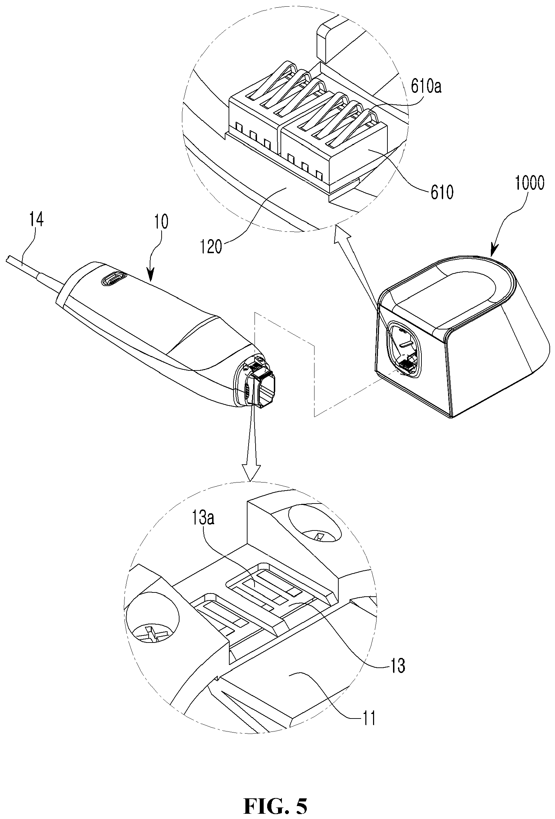

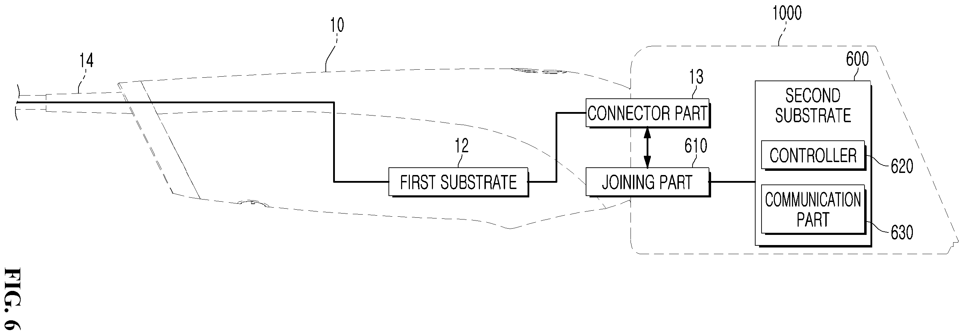

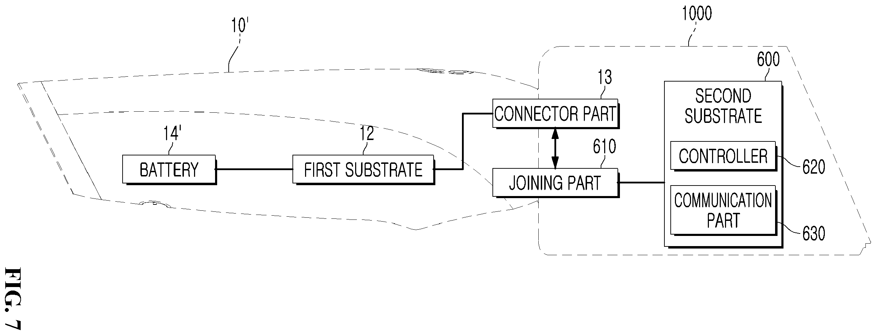

FIG. 1 is a perspective view of an oral scanner system according to one embodiment of the present disclosure. FIGS. 2 and 3 are exploded perspective views of an oral scanner calibration device according to one embodiment of the present disclosure. FIG. 4 is a lateral cross-sectional view of the oral scanner calibration device according to one embodiment of the present disclosure. FIG. 5 shows enlarged views of the oral scanner system according to one embodiment of the present disclosure. FIG. 6 is a schematic diagram of the oral scanner system according to one embodiment of the present disclosure. FIG. 7 is a schematic diagram of the oral scanner system according to another embodiment of the present disclosure.

As shown in FIGS. 1 to 3, an oral scanner system 1 according to one embodiment of the present disclosure includes an oral scanner body 10 and an oral scanner calibration device 1000.

Generally, an oral scanner includes the oral scanner body 10 having an optical device and an imaging board disposed thereon and a probe tip having a reflector disposed therein, and the optical device includes a light source configured to irradiate light toward an opening of the oral scanner body 10 along a central axis of the oral scanner body 10 and a pair of imaging devices configured to condense light introduced through the opening to the imaging board.

Here, since oral scanning of a patient is performed while the probe tip is inserted into the oral cavity of the patient, a reflective member may get damp due to moisture in the oral cavity of the patient, and thus, in the case in which calibration is performed while the probe tip is mounted, accuracy of calibration may be degraded. Only the oral scanner body 10 from which the probe tip is removed may be inserted into the oral scanner calibration device 1000.

The oral scanner calibration device 1000 according to one embodiment of the present disclosure is a device for calibrating an optical device of an oral scanner and includes a housing 100, a pattern plate part 200, and a reflector part 300.

The housing 100 according to one embodiment of the present disclosure forms an outer peripheral surface of the oral scanner calibration device 1000 and includes a body 110 forming an inner space, an insertion part 120 formed at one side of a front of the body 110 and into which the oral scanner body 10 is inserted to be supported, and a first cover 130 and a second cover 140 formed to cover the top and bottom of the body 110. A first support part 141 and a second support part 142 that may axially support a driving part 400 and a rotating part 500 which will be described below are positioned at the second cover 140 of the housing 100.

According to one embodiment of the present disclosure, the insertion part 120 may be formed to correspond to the shape of a connecting part 11 which is at an end of the oral scanner body 10 for the oral scanner body 10 to be firmly fixed to the insertion part 120.

Meanwhile, the pattern plate part 200 according to one embodiment of the present disclosure is for calibrating the oral scanner while positioned inside the housing 100 and includes a pattern plate 210 and a seating part 220.

Specifically, the pattern plate 210 may be formed in a square shape and may have various patterns formed thereon. For example, a pattern may be formed so that, in a grid pattern, light and shade are clearly distinguished between grids adjacent in up-down and left-right directions.

Meanwhile, an angle of inclination of the pattern plate 210 according to one embodiment of the present disclosure may be set to be 40° or more and less than 50° based on incident light. In the case in which the pattern plate 210 is disposed to be orthogonal to the incident light, there is a disadvantage that each of the patterns formed on the pattern plate 210 has the same depth information (or height information) for the same surface. Thus, the pattern plate 210 is designed to be disposed to be inclined at a predetermined angle relative to incident light to increase an effect of calibration.

To this end, a front of the seating part 220 on which the pattern plate 210 is seated may be formed of an inclined surface 221 that forms an angle of inclination of 40° or more and less than 50° relative to an optical axis of incident light.

Meanwhile, as shown in FIG. 3, the seating part 220 according to one embodiment of the present disclosure may include an extension 222 extending to a rear of the inclined surface 221 along the optical axis of the incident light. The extension 222 is integrally coupled to a first rotating part 530 of the rotating part 500 which will be described below, and thus, the pattern plate part 200 rotates in conjunction with rotation of the rotating part 500.

Meanwhile, due to being positioned to be spaced a predetermined distance apart from the central axis of the oral scanner body 1, the pattern plate part 200 according to one embodiment of the present disclosure may face the optical device inside the oral scanner body 1 through the reflector part 300.

Specifically, in order to reflect light irradiated from the optical device of the oral scanner body 10 toward the pattern plate part 200, the reflector part 300 may include a plurality of reflectors 310 and 320 and a support part 330 configured to support the reflectors 310 and 320 for the reflectors 310 and 320 to be inclined relative to the oral scanner body 10.

More specifically, the support part 330 includes a first inclined surface 331 formed to be inclined at an angle of, for example, 45° relative to the central axis of the oral scanner body 10 and a second inclined surface 332 formed to be inclined at an angle of, for example, 45° relative to the optical axis of incident light incident on the pattern plate part 200. A first reflector 310 is disposed on the first inclined surface 331, and a second reflector 320 is disposed on the second inclined surface 332. That is, a plane including the first inclined surface 331 and a plane including the second inclined surface 332 may be disposed to be orthogonal to each other.

In this case, light irradiated from the optical device may be reflected 180° through the first reflector 310 and the second reflector 320 and incident on the pattern plate 210. Here, the reflectors may be formed as rectangular mirrors but are not limited thereto, and of course, mirrors of various other shapes may be applied.

Meanwhile, the reflector part 300 according to one embodiment of the present disclosure may include a guide part 340 to linearly move along a guide rail 143 in conjunction with rotation of the rotating part 500 which will be described below.

Specifically, the guide rail 143 is provided on an inner side surface of the second cover 140 and longitudinally extends in a direction parallel to the central axis of the oral scanner body 10. Further, the guide part 340 is positioned at one side of a lower end of the support part 330, and a guide groove 341 corresponding to the guide rail 143 is formed on a bottom surface of the guide part 340. Here, when the guide rail 143 is seated on the guide groove 341, as will be described below, the reflector part 300 may linearly move in the longitudinal direction of the guide rail 143 during rotation of the rotating part 500.

Meanwhile, as described above, in an oral scanner using a stereo vision method, an image of one point of an oral cavity is captured by two or more imaging devices to obtain two or more pieces of image data, and 3D distance information is obtained based on the two or more pieces of image data, and thus calibration is frequently required for the oral scanner in order to obtain accurate 3D model data. Here, when an image of the pattern plate part is obtained from various angles and depths, accuracy of calibration can be improved.

To this end, the oral scanner calibration device 1000 according to one embodiment of the present disclosure may further include the driving part 400 and the rotating part 500.

The driving part 400 according to one embodiment of the present disclosure provides a driving force to the rotating part 500 and may include a driving motor 410, a driving gear 420, and a driving belt 430. The driving motor 410 may be supported on the above-mentioned first support part 141 of the housing 100, and the driving gear 420 in the shape of a pulley and the driving belt 430 surrounding an outer circumferential surface of the driving gear 420 may be provided at an end of a driving shaft of the driving motor 410. Here, a driving method of the driving part 400 is not limited to the above-described belt driving method, and various other driving methods may be applied.

Meanwhile, the oral scanner calibration device 1000 according to one embodiment of the present disclosure may further include a controller 620. The controller 620 checks a control signal received from an image processing device such as a personal computer (PC) or the oral scanner calibration device 1000 and controls operation performance of the driving part 400.

Meanwhile, the oral scanner calibration device 1000 according to one embodiment of the present disclosure may further include a communication part 630. The communication part 630 is in charge of communication between the oral scanner calibration device 1000 and an image processing device or between the oral scanner calibration device 1000 and the oral scanner body 10 and transmits a control signal received from the image processing device or the oral scanner body 10 to the controller 620. The communication part 630 is capable of both wired connection through a universal serial bus (USB) or the like and wireless connection through Bluetooth, Wi-Fi, or the like.

Meanwhile, the rotating part 500 according to one embodiment of the present disclosure receives a driving force from the driving part 400 and rotates in conjunction with the pattern plate part 200 and the reflector part 300 and may include a rotating shaft 510, a power transmitter 520, the first rotating part 530, and a second rotating part 540.

Specifically, the rotating shaft 510 is in the shape of a rod that extends in the direction parallel to the central axis of the oral scanner body 10, and both ends of the rotating shaft 510 may be axially supported by the first support part 141 and the second support part 142 of the housing 100 so that the rotating shaft 510 is rotatable. According to one embodiment of the present disclosure, a bearing that facilitates rotation of the rotating shaft 510 may be provided inside the first support part 141 and the second support part 142.

The power transmitter 520 configured to receive the driving force of the driving part 400 is positioned at one end of the rotating shaft 510 according to one embodiment of the present disclosure. The power transmitter 520 may be formed in the shape of a pulley for the driving belt 430 of the driving part 400 to be wound around the power transmitter 520, but the shape of the power transmitter 520 is not limited thereto, and gears of various other shapes may be applied according to the driving method of the driving part 400.

The first rotating part 530 may be positioned at the other end of the rotating shaft 510 according to one embodiment of the present disclosure. According to one embodiment of the present disclosure, the first rotating part 530 may include a plurality of gears. For example, as shown in FIG. 3, the first rotating part 530 may include a first gear 531 positioned at the other end of the rotating shaft 510 and a second gear 532 engaged with the first gear 531 at an upper side of the first gear 531. Here, the first gear 531 and the second gear 532 may be axially supported by the second support part 142. The first gear 531 and the second gear 532 may each be formed in the shape of a spur gear but are not limited thereto, and of course, gears of various other shapes may be applied.

According to one embodiment of the present disclosure, the second gear 532 may be engaged with the extension 222 of the pattern plate part 200. Thus, when the rotating shaft 510 rotates, the pattern plate part 200 rotates in place about the optical axis of incident light.

According to one embodiment of the present disclosure, the second rotating part 540 may be positioned at one side of the rotating shaft 510. For example, as shown in FIG. 4, the second rotating part 540 may include screw threads provided on an outer circumferential surface of the rotating shaft 510. The screw threads of the second rotating part 540 may be coupled to the support part 330 of the reflector part 300 so that the second rotating part 540 and the reflector part 300 are rotatable relative to each other.

Meanwhile, since the reflector part 300 may rotate along the rotating shaft 510 due to a frictional force acting between the second rotating part 540 and the support part 330 when the rotating shaft 510 rotates, the guide part 340 may be seated on the guide rail 143 formed on the second cover 140 of the housing 100. In this case, rotation of the guide part 340 is restricted due to the guide rail 143 being positioned within a radius of rotation of the guide part 340, and the guide part 340 linearly moves in the longitudinal direction of the guide rail 143.

That is, the reflector part 300 linearly moves relative to the oral scanner body 10 in conjunction with the rotation of the rotating part 500.

According to one embodiment of the present disclosure, a step part 511 protruding in a radial direction of the rotating shaft 510 may be formed at both ends of the second rotating part 540 to limit a range of linear movement of the pattern plate part 200.

Meanwhile, in the related art, since a power source is separately provided for each of an oral scanner calibration device and an oral scanner, it is inefficient in terms of energy, and there are limitations in reducing the weight and volume of the oral scanner calibration device.

Thus, according to one embodiment of the present disclosure, upon insertion of the oral scanner body 10 into the oral scanner calibration device 1000, the oral scanner calibration device 1000 is electrically connected to the oral scanner body 10 and receives power.

Specifically, as shown in FIGS. 5 and 6, the oral scanner body 10 includes a first substrate 12 therein, and a cable 14 connected to an external power source is joined to the first substrate 12 to supply power to the oral scanner body 10.

However, the present disclosure is not limited thereto, and as shown in FIG. 7, an oral scanner body 10' according to another embodiment of the present disclosure may include a battery 14' positioned therein. In this case, the battery 14' may be electrically connected to the first substrate 12 and supply power to the oral scanner body 10'.

Further, a connector part 13 that can be connected to a joining part 610 which will be described below is provided on the first substrate 12. Specifically, joining terminals of the connector part 13 may be formed of a plurality of conductive lines and, according to one embodiment of the present disclosure, may be formed as grooves 13a exposed to the outside of the connecting part 11 which is at the end of the oral scanner body 10.

The oral scanner calibration device 1000 includes a second substrate 600 positioned inside the housing 100. The controller 620 and the communication part 630 are mounted on the second substrate 600, and the joining part 610 that can be electrically joined to the connector part 13 is provided.

Specifically, joining terminals of the joining part 610 may be formed of a plurality of conductive lines, and according to one embodiment of the present disclosure, the joining part 610 may be formed of a plurality of pins 610a protruding to an inner circumferential surface of the insertion part 120. In this case, upon insertion of the oral scanner body 10 into the insertion part 120, the pins 610a may be inserted into the grooves 13a.

That is, upon insertion of the oral scanner body 10 into the insertion part 120 of the oral scanner calibration device 1000, the connector part 13 of the oral scanner body 10 may be electrically connected to the joining part 610 of the oral scanner calibration device 1000, and power may be supplied to the oral scanner calibration device 1000.

In this case, the communication part 630 of the oral scanner calibration device 1000 may be connected to an image processing device or the oral scanner body 10 to communicate therewith.

Here, a rigid printed circuit board (PCB) in which a circuit is configured by printing copper (Cu) on an epoxy or phenolic resin or a flexible printed circuit board (FPCB) in which various circuit patterns are formed by copper (Cu), gold (Au), or other conductive materials on a polyamide film with excellent flexibility may be used as the above-described first substrate 12 and second substrate 600, but the present disclosure is not limited thereto.

Hereinafter, a process in which the oral scanner system 1 according to one embodiment of the present disclosure performs calibration will be described in detail.

Upon insertion of the oral scanner body 10 into the insertion part 120, the connector part 13 and the joining part 610 electrically come in contact, and power may be supplied to the controller 620 and the communication part 630. Then, when a driving signal is transmitted to the driving part 400 by the controller 620, the pattern plate part 200 rotates in place about the optical axis of incident light, and the reflector part 300 linearly moves relative to the oral scanner body 10. In this way, an image of the pattern plate part 200 can be obtained from various angles and depths, and thus accuracy of calibration can be improved.

That is, according to the oral scanner system 1 according to one embodiment of the present disclosure, since it is possible to supply power of the oral scanner body 10 to the oral scanner calibration device 1000 by inserting the oral scanner body 10 into the oral scanner calibration device 1000, it is efficient in terms of energy, and size reduction of the oral scanner calibration device 1000 can be achieved.

The above-given description of the present disclosure is only illustrative, and those of ordinary skill in the art to which the present disclosure pertains should understand that the present disclosure may be easily modified to other specific forms without changing the technical spirit or essential features of the present disclosure. Therefore, the embodiments described above should be understood as illustrative, instead of limiting, in all aspects. For example, each element described as a single type may be embodied in a distributed manner, and likewise, elements described as being distributed may be embodied in a combined form.

The scope of the present disclosure is shown by the claims below, and all changes or modifications derived from the meaning and scope of the claims and their equivalent concepts should be construed as falling within the scope of the present disclosure.

### (Description of reference numerals)

1: oral scanner system
10: oral scanner body, 11: connecting part, 12: first substrate, 13: connector part, 13a: groove, 14: cable
10': oral scanner body, 14': battery
1000: oral scanner calibration device
100: housing, 110: body, 120: insertion part, 130: first cover, 140: second cover, 141: first support part, 142: second support part, 143: guide rail
200: pattern plate part, 210: pattern plate, 220: seating part, 221: inclined surface, 222: extension
300: reflector part, 310: first reflector, 320: second reflector, 330: support part, 331: first inclined surface, 332: second inclined surface, 340: guide part, 341: guide groove
400: driving part, 410: driving motor, 420: driving gear, 430: driving belt
500: rotating part, 510: rotating shaft, 511: step part, 520: power transmitter, 530: first rotating part, 531: first gear, 532: second gear, 540: second rotating part
600: second substrate, 610: joining part, 610a: pin, 620: controller, 630: communication part

## Claims

1. An oral scanner system comprising:
an oral scanner body which has an optical device and an imaging board disposed therein and is configured to receive power from an external power source or an internal battery; and
an oral scanner calibration device configured to support the oral scanner body and calibrate the optical device using a pattern plate provided therein,
wherein, upon insertion of the oral scanner calibration device into the oral scanner body, the oral scanner calibration device is electrically connected to the oral scanner body, and power is supplied to the oral scanner calibration device.

2. The oral scanner system of claim 1, wherein:
the oral scanner body includes a first substrate electrically connected to the external power source or the internal battery and a connector part mounted on the first substrate; and
the oral scanner calibration device includes a second substrate and a joining part mounted on the second substrate.

3. The oral scanner system of claim 1, wherein the oral scanner calibration device includes a casing in which an insertion part, into which the oral scanner body is inserted, is formed.

4. The oral scanner system of claim 3, wherein, upon insertion of the oral scanner body into the insertion part, the connector part and the joining part are electrically connected.

5. The oral scanner system of claim 4, wherein:
the connector part is formed of a groove including a conductive line;
the joining part is formed of a pin including a conductive line; and
upon the insertion of the oral scanner body into the insertion part, the pin is inserted into the groove.

6. The oral scanner system of claim 3, wherein:
the connector part is positioned at an end of the oral scanner body; and
the joining part is positioned at the insertion part.

7. The oral scanner system of claim 3, wherein the insertion part is formed to correspond to a shape of the end of the oral scanner body.

8. The oral scanner system of claim 1, wherein the oral scanner calibration device includes a driving part configured to be driven to change one or more of an angle and a distance of the pattern plate relative to the optical device.

9. The oral scanner system of claim 8, wherein the oral scanner calibration device includes a controller configured to control operation performance of the driving part.

10. The oral scanner system of claim 9, wherein the oral scanner calibration device includes a communication part configured to communicate with an image processing device or the oral scanner body and transmit a control signal to the controller.
